(19) Europäisches Patentamt
European Patent Office
Office européen des brevets

(11) Publication number: **0 492 391 A2**

(12) **EUROPEAN PATENT APPLICATION**

(21) Application number: **91121689.3**

(51) Int. Cl.5: **A61K 7/06**, A61K 7/48

(22) Date of filing: **18.12.91**

(30) Priority: **26.12.90 JP 406614/90**

(43) Date of publication of application: **01.07.92 Bulletin 92/27**

(84) Designated Contracting States: **AT BE CH DE DK ES FR GB GR IT LI LU MC NL SE**

(71) Applicant: **NISSAN CHEMICAL INDUSTRIES LTD.**
**3-7-1, Kanda Nishiki-cho**
**Chiyoda-ku Tokyo(JP)**

(72) Inventor: **Yamashita, Toru, c/o Nissan Chemical Ind., Ltd.**
**1470, Ohaza Shiraoka, Shiraoka-cho**
**Minamisaitama-gun, Saitama-ken(JP)**
Inventor: **Masuda, Yukinori, c/o Nissan Chemical Ind., Ltd.**
**1470, Ohaza Shiraoka, Shiraoka-cho**
**Minamisaitama-gun, Saitama-ken(JP)**
Inventor: **Seto, Kiyotomo, c/o Nissan Chemical Ind., Ltd.**
**Central Research Center, 722-1, Tsuboi-cho**
**Funabashi-shi, Chiba-ken(JP)**

(74) Representative: **Hansen, Bernd, Dr. Dipl.-Chem. et al**
**Hoffmann, Eitle & Partner Patent- und Rechtsanwälte Arabellastrasse 4 Postfach 81 04 20**
**W-8000 München 81(DE)**

(54) **Hair growth stimulator.**

(57) A hair growth stimulating agent or alopecia remedy comprising as an active agent a compound of the formula (I):

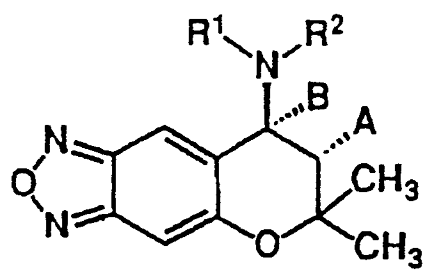

I

wherein A represents OH or $OC(O)CH_{3-n}X_n$ or forms a bond together with B;

B represents hydrongen atom or forms a bond together with A;

if $R^1$ is hydrogen atom, $R^2$ represents hydrogen atom, $C(Z)CH_{3-n}X$ or $C(Z)NHCH_{3-n}X_n$, or

if $R^1$ is not hydrogen atom, $R^1$ and $R^2$ together form $(CH_2)_m$, $(CH_2)_{m-1}C(Z)$, $N(R^3)(CH_2)_2C(Z)$, $(CH_2)_{m-2}NHC(Z)$ or $(CH^2)_{m-2}OC(Z)$;

X represents fluorine, chlorine, bromine atom, methyl group, and methoxy group;

Z represents oxygen or sulfur atom;

$R^3$ represents hydrogen atom or methyl group;

m is 4 or 5

n is 0 or an integer of 1-3,

or a pharmaceutically acceptable salt of the compound which can form salt.

EP 0 492 391 A2

## BACKGROUND OF THE INVENTION

The present invention relates to a hair growth stimulating agent or alopecia remedy comprising a pyranobenzoxadiazole derivative as an active ingredient and a method for preparing it.

EP-A-0327127 and U.S. Patent No. 4900752 disclose that a pyranobenzoxadiazole derivative will be usefull for the therapy of circulatory diseases such as hypertension, angina pectoris, and arrhythmia because of its ability to highly activate the K-channel. However, they mention nothing about using it as a hair growth stimulator or alopecia remedy.

It is suggested that a benzopyran derivative, which is known to activate the K-channel, will be effective for alopecia therapy (WO8800-822-A). However, nothing is known about the relation between both actions.

Everybody suffers more from alopecia (such as androgenic alopecia, male pattern baldness, and alopecia premature) as he grows older. Alopecia results from malnutrition, endocrinopathy, autonomic imbalance, and nervous stress. Various attempts have been made for alopecia therapy with hormones, vitamins, crude drugs, amino acids, scalp stimulants, anti-inflammatory drugs, etc.; but they are not successful so far.

The present inventors carried out extensive studies in search of compounds which are effective in hair growth stimulation or alopecia therapy. It was found that a specific pyranobenzoxadiazole greatly stimulates the growth of hair. This finding led to the present invention.

## SUMMARY OF THE INVENTION

It is an object of the present invention to provide a hair growth stimulating agent or alopecia remedy.

This object is solved by providing a hair growth stimulating agent or alopecia remedy comprising as an active agent a compound of the formula (I)

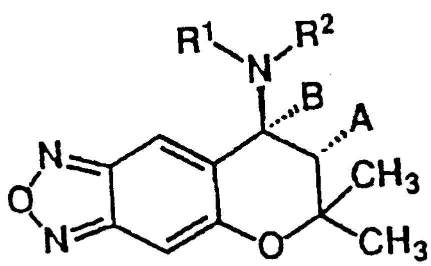

(I)

wherein A represents OH or $OC(O)CH_{3-n}X_n$ or forms a bond together with B;

B represents hydrogen atom or forms a bond together with A;

and if $R^1$ is hydrogen atom, $R^2$ represents hydrogen atom, $C(Z)CH_{3-n}X_n$, or $C(Z)NHCH_{3-n}X_n$ or

if $R^1$ is not hydrogen atom, $R^1$ and $R^2$ together form $(CH_2)_m$, $(CH_2)_{m-1}C(Z)$, $N(R^3)(CH_2)_2C(Z)$, $(CH_2)_{m-2}NHC(Z)$, or $(CH_2)_{m-2}OC(Z)$;

X represents fluorine, chlorine or bromine atom, methyl group or methoxy group;

Z represents oxygen or sulfur atom;

$R^3$ represents hydrogen atom or methyl group;

m is 4 or 5;

n is 0 or an integer of 1 to 3,

or a pharmaceutically acceptable salt of the compound which can form salt. It is produced by the process disclosed in EP-A 0327127 and U.S. Patent No. 4900752.

Having an asymmetric center, the compound used in the present invention embraces optical isomers, which are within the scope of the present invention. The optical isomers are produced by the process disclosed in Japanese Patent Laid-open No. Hei 3-141286(141286/1991) and EP-A-409165.

The compound of the present invention can be administered orally or parenterally as it is, or as appropriate forms of pharmaceutical compositions. The pharmaceutical compositions include oral agents such as tablets, medicated syrups, parenteral agents such as ointments, creams, lotions, pastes, jellies, gels, mousses, and aerosols.

A hair growth stimulating agent or alopecia remedy is prepared by mixing the active ingredient with carriers and/or diluents.

According to the present invention, the content of the active ingredient is 0.005-10%, preferably 0.05-3% and the dose of the active ingredient is 0.001-100 mg/day, preferably 0.01-10 mg/day, (once to several

times a day) depending on the symptom, weight, age, and sex of the patient.

Preferable compounds to be used in the present invention varies depending on whether $R^1$ of the formula (I) is hydrogen atom or not. If $R^1$ is hydrogen atom, $R^2$ is preferably $COCH_3$, $COCH_2CH_3$, $COCH_2F$, $COCF_3$, $COCH_2Cl$, or $CONHCH_3$. If $R^1$ is not hydrogen atom, $R^1$ and $R^2$ together form $-(CH_2)_3CO-$ , and $-(CH_2)_4CO-$, $-(CH_2)_3OCO-$, $-NH(CH_2)_2CO-$, $-(CH_2)_4-$ or $-(CH_2)_5-$, of which $-(CH_2)_3CO-$ and $-(CH_2)_4CO$ are preferable. Especially, compounds of the formulas (II), (III) and (IV) are more preferable.

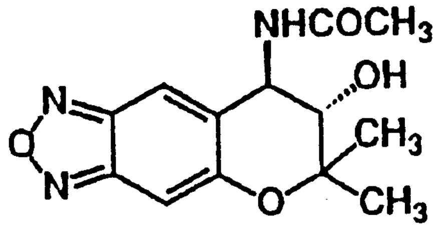

(II)

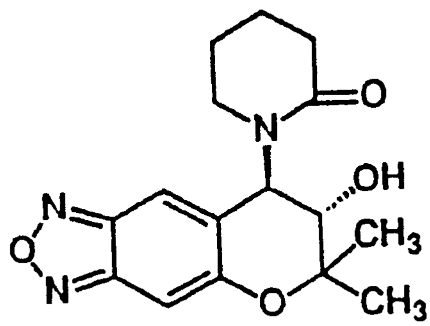

(III)

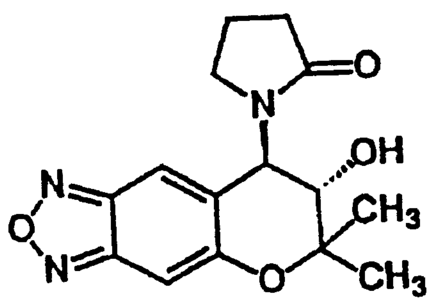

(IV)

These compounds have a low level of toxicity and hence are usable as pharmaceutical preparations, as disclosed in EP-A 0327127 and U.S. Patent No. 4900752.

The invention will be described with reference to the following examples, which are not intended to restrict the scope of the invention.

Test Example

A 0.5% solution in ethanol/water mixture (1/1) of the compound of the formula (III) was locally applied at a dose of 50 µL once a day to 10 male mice (ICR strain), with their hack depilated by a depilatory cream ("Evercream S" made by Tokyo Tanabe Co., Ltd.).

The hair growing effect was evaluated by comparing the hair length with that of the control group (treated with 1:1 ethanol/water mixture) on the 14th day and 21st day after the start of application. The results are shown in Table 1. It is clearly noted that the compound stimulated the growth of hair.

Table 1

| Hair length (mm) | | |
|---|---|---|
| | 14th day | 21st day |
| Control group | 3.15±0.13 | 7.39±0.14 |
| Treated group | 3.76±0.25* | 8.94±0.16** |

*P < 0.05,
** P < 0.01

| Formulation Example 1 (tablets) | |
|---|---|
| Compound of the formula (III) | 10 g |
| Lactose | 260 g |
| Microcrystalline cellulose | 600 g |
| Corn starch | 350 g |
| Hydroxypropyl cellulose | 100 g |
| CMC-Ca (carboxymethylcellulose calcium) | 150 g |
| Magnesium stearate | 30 g |
| Total | 1500 g |

The above components were mixed by a usual method and the mixture was formed into 10,000 tablets, each containing 1 mg of the active ingredient.

| Formulation Example 2 (capsules) | |
|---|---|
| Compound of the formula (III) | 10 g |
| Lactose | 440 g |
| Microcrystalline cellulose | 1000 g |
| Magnesium stearate | 50 g |
| Total | 1500 g |

The above components were mixed by a usual method and the mixture was filled into 10,000 gelatin capsules, each containing 1 mg of the active ingredient.

| Formulation Example 3 (ointment) | |
|---|---|
| Compound represented by the formula (III) | 1.0 g |
| Liquid paraffin | 10.0 g |
| Cetanol | 20.0 g |
| White vaseline | 68.4 g |
| Ethylparaben | 0.1 g |
| ℓ-menthol | 0.5 g |
| Total | 100.0 g |

The above components were mixed by a usual method to obtain a 1% ointment.

| Formulation Example 4 (liquid solution) | |
|---|---|
| Compound of the formula (III) | 5 g |
| Propylene glycol | 205 g |
| Ethyl alcohol | 790 g |
| Total | 1000 g |

The above components were mixed by a usual method to obtain 0.5% liquid solution.

| Formulation Example 5 (Aerosol) | |
|---|---|
| Compound of the formula (III) | 1 g |
| Perfume | 1 g |
| Ethyl alcohol | 398 g |
| Propellant (Freon 12/11, 80:20) | 600 g |
| Total | 1000 g |

The above components were mixed by a usual method to obtain a 0.1% aerosol.

## Claims

**1.** A hair growth stimulating agent or alopecia remedy comprising as an active ingredient a compound of the formula (I):

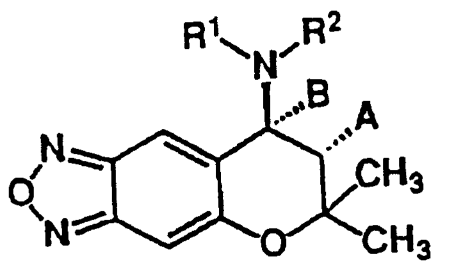

(I)

wherein A represents OH or $OC(C)CH_{3-n}X_n$ or forms a bond together with B;

B represents hydrogen atom or forms a bond together with A;

if $R^1$ is hydrogen atom, $R^2$ represents any of hydrogen atom, $C(Z)CH_{3-n}X_n$ or $C(Z)NHCH_{3-n}X_n$, or

if $R^1$ is not hydrogen atom, $R^1$ and $R^2$ together form $(CH_2)_m$, $(CH_2)_{m-1}C(Z)$, $N(R^3)(CH_2)_2C(Z)$, $(CH_2)_{m-2}NHC(Z)$ or $(CH_2)_{m-2}OC(Z)$;

X represents fluorine, chlorine, bromine atom, methyl group, or methoxy group;

Z represents oxygen or sulfur atom;

$R^3$ represents hydrogen atom or methyl group;

m is 4 or 5;

n is 0 or an integer of 1-3,

or a pharmaceutically acceptable salt of the compound which can form salt.

**2.** A hair growth stimulating agent or alopecia remedy according to claim 1 wherein $R^1$ represents hydrogen atom and $R^2$ represents $CH_3CO$, $CH_3CH_2CO$, or $CH_3NHCO$.

**3.** A hair growth stimulating agent or alopecia remedy as claimed in claim 1 wherein $R^1$ and $R^2$ in the formula (I) together form $-(CH_2)_4CO-$, $-(CH_2)_3CO-$, $-(CH_2)_3OCO-$, or $-NH(CH_2)_2CO-$.

**4.** A hair growth stimulating agent or alopecia remedy according to claim 1 wherein the active ingredient is a compound of the formula (II):

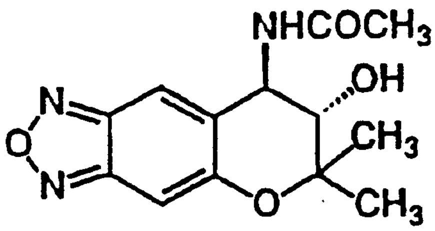

(II)

5. A hair growth stimulating agent or alopecia remedy according to claim 1 which comprises as an active ingredient a compound of the formula (III):

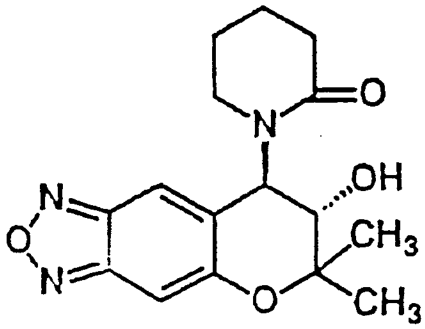

(III)

6. A hair growth stimulating agent or alopecia remedy according to claim 1 which comprises as an active ingredient a compound of the formula (IV):

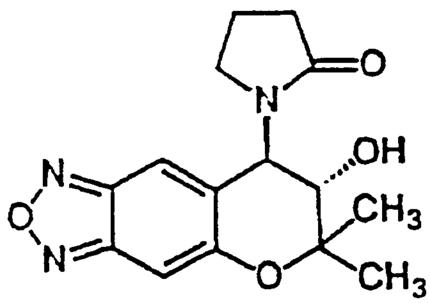

(IV)

7. A method for preparing a hair growth stimulating agent or alopecia remedy by mixing an active ingredient as defined in any of claims 1 to 6 which carriers and/or diluents.

8. Use of an active ingredient as defined in any of the claims 1 to 6 for the preparation of a hair growth stimulating agent or alopecia remedy.

**Claims for the following Contracting States : GR, ES**

1. A method for preparing a hair growth stimulating agent or alopecia remedy by mixing a compound of the formula (I) as an active ingredient:

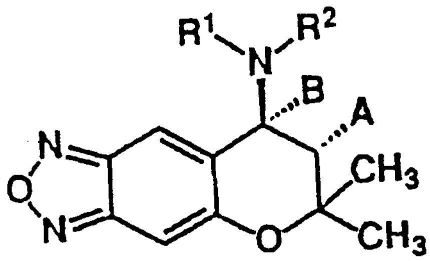

(I)

wherein A represents OH or $OC(O)CH_{3-n}X_n$ or forms a bond together with B;

B represents hydrogen atom or forms a bond together with A;

if $R^1$ is hydrogen atom, $R^2$ represents any of hydrogen atom, $C(Z)CH_{3-n}X_n$ or $C(Z)NHCH_{3-n}X_n$, or

if $R^1$ is not hydrogen atom, $R^1$ and $R^2$ together form $(CH_2)_m$, $(CH_2)_{m-1}C(Z)$, $N(R^3)(CH_2)_2C(Z)$, $(CH_2)_{m-2}NHC(Z)$ or $(CH_2)_{m-2}OC(Z)$;

X represents fluorine, chlorine, bromine atom, methyl group, or methoxy group;

Z represents oxygen or sulfur atom;

$R^3$ represents hydrogen atom or methyl group;

m is 4 or 5;

n is 0 or an integer of 1-3,

or a pharmaceutically acceptable salt of the compound which can form salt.

with carriers and/or diluents.

**2.** A method as claimed in claim 1 wherein $R^1$ represents hydrogen atom and $R^2$ represents $CH_3CO$, $CH_3CH_2CO$, or $CH_3NHCO$.

**3.** A method as claimed in claim 1 wherein $R^1$ and $R^2$ in the formula (I) together form $-(CH_2)_4CO-$, $-(CH_2)_3CO-$, $-(CH_2)_3OCO-$, or $-NH(CH_2)_2CO-$.

**4.** A method as claimed in claim 1 which comprises using as an active ingredient a compound of the formula (II):

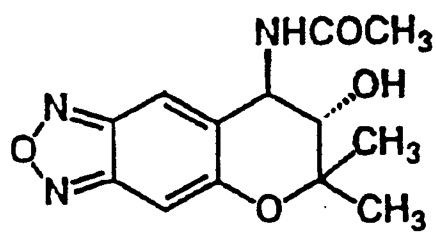

(II)

**5.** A method as claimed in claim 1 which comprises using as an active ingredient a compound of the formula (III):

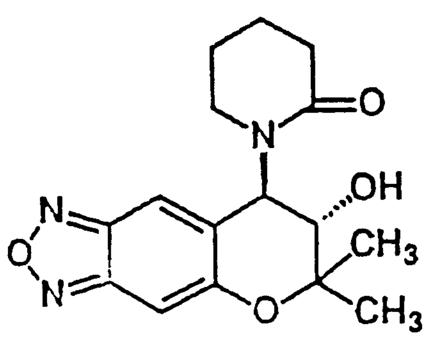

(III)

**6.** A method as claimed in claim 1 which comprises using as an active ingredient a compound of the formula (IV):

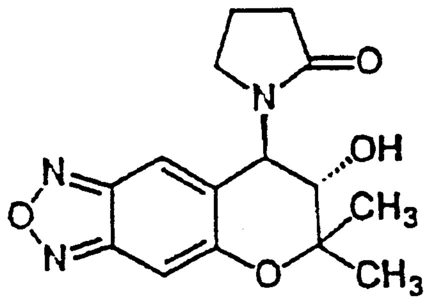

(IV)

**7.** Use of an active ingredient as defined in any of the claims 1 to 6 for the preparation of a hair growth stimulating agent or alopecia remedy.